# NEUE EUROPÄISCHE PATENTSCHRIFT

(11) **EP 0 421 473 B2**
(45) Veröffentlichungstag und Bekanntmachung des Hinweises auf die Entscheidung über den Einspruch: **02.02.2000**
(45) Hinweis auf die Patenterteilung: 02.04.1997
(21) Anmeldenummer: 90119183.3
(22) Anmeldetag: 05.10.1990
(51) Int. Cl.: A61F 13/15, A61F 5/44

(54) **Wegwerfwindel**
Disposable diaper
Couche à jeter

(30) Priorität: 05.10.1989 JP 26183789
(43) Veröffentlichungstag der Anmeldung: 10.04.1991
(73) Patentinhaber: UNI-CHARM CORPORATION, Kawanoe-shi Ehime-ken (JP)
(72) Erfinder: Yamamoto, Masmitsu, Kawanoe-shi Ehime-ken (JP); Kimura, Noriyuki 5-8-70 Chuo, Ehime-ken (JP); Fujioka,Yosihisa, Mitoyo-gun Kagawa-ken (JP); Yamamoto, Hiroki, Kawanoe-shi,Ehime-ken (JP); Suekane, Makoto, Kawanoe-shi, Ehime-ken (JP)
(74) Vertreter: Sperling, Rüdiger, Dipl.-Ing.

(56) Entgegenhaltungen:
- EP-A- 0 109 126
- EP-A- 0 190 881
- EP-A- 0 269 401
- FR-A- 2 577 115
- FR-A- 2 585 217
- US-A- 4 585 448
- US-A- 4 887 602

## Beschreibung

### HINTERGRUND DER ERFINDUNG

Die Erfindung bezieht sich auf Wegwerfwindeln, die ausgelegt sind, Ausscheidungen aufzunehmen, insbesondere solche Wegwerfwindeln oder Unterhosen, die für erwachsene Benutzer geeignet sind.

Die meisten üblicherweise erhältlichen Wegwerfwindeln für erwachsene Benutzer sind durch eine im wesentlichen T-förmige Konfiguration ihrer Gesamtgestalt gekennzeichnet, die durch einen rückwärtigen Abschnitt und einem Paar von Bauchabschnitten definiert wird, die sich seitlich von einander gegenüberliegenden Seiten des rückwärtigen Abschnitts erstrecken.

Windeln einer derartigen Konfiguration sind allgemein in zwei Typen Klassifiziert und zwar hinsichtlich des Ablaufs des Anlegens, wenn der Benutzer im Bett liegt.

Bei dem ersten Typ legt eine Pflegeperson einen rückwärtigen Abschnitt einer Windel auf die rückwärtige Hüfte des Trägers, plaziert einen Schrittabschnitt der Winde in den Schritt des Trägers, wobei der vordere Abschnitt der Windel noch in der Hand gehalten wird. Anschließend wird der vordere Abschnitt auf den Bauch des Trägers gelegt und schließlich die Bauchabschnitte über den Magen des Benutzers geklappt und befestigt. Die meisten der konventionellen Windeln sind von diesem ersten Typ, soweit es die Sequenz des Anlegens betrifft.

Bei dem zweiten Typ, wie er z.B aus der FR-A-2,585,217 bekannt ist legt eine Pflegeperson den rückwärtigen Abschnitt einer Windel auf die rückwärtige Hüfte des Trägers, befestigt deren Bauchabschnitte über dem Bauch des Trägers miteinander, legt den Schrittabschnitt auf den Schritt des Trägers, wobei ein vorderer Abschnitt davon noch in der Hand gehalten wird, anschließend wird dieser vordere Abschnitt auf den Bauch des Trägers gelegt, um die Bauchabschnitte zu überdecken und schließlich wird dieser vordere Abschnitt auf den Bauchabschnitten befestigt. Hinsichtlich des leichten Anlegens der Windel ist die letztere besser als erstere, da beim letzteren Typ die Windel um die Hüfte des Trägers herum mit einem Zwischenschritt beim Anlegen durch Verbinden der beiden Bauchabschnitte miteinander über dem Bauch des Trägers befestigt wird und die Windel vom Träger selber angelegt werden kann, wenn er aufsteht.

Beim letzteren Typ kann jedoch ein in dem vorderen Abschnitt angeordneter Saugkern nicht wirksam zum Aufsaugen von flüssigen Ausscheidungen verwendet werden, da eine flüssigkeitsundurchlässige Rücklage sich über die gesamte Windel einschließlich Seitenabschnitte hinweg erstreckt. Im einzelnen wird der vordere Abschnitt auf die Bauchabschnitte mit der flüssigkeitsundurchlässigen rückwärtigen Lage gelegt und diese Bauchabschnitte bilden eine Barriere gegen den Durchtritt von flüssigen Ausscheidungen zu dem vorderen Abschnitt hin. Wenn nun der vordere Abschnitt für das Aufsaugen der flüssigen Ausscheidungen in dieser Weise nicht vollständig verwendet werden kann, ist die Fähigkeit Flüssigkeit aufzusaugen in dem vorderen Abschnitt ungenügend, um ein unerwünschtes Auslaufen von flüssigen Ausscheidungen, insbesondere, wenn es sich bei dem Träger um einen Erwachsenen handelt, zu vermeiden. Ein Auslaufen aufgrund solcher ungenügender Flüssigkeitssaugfähigkeit im vorderen Abschnitt tritt insbesondere dann auf, wenn es sich bei dem Träger um eine männliche Person handelt oder im Falle einer weiblichen Person, wenn diese im Bett auf der Seite liegt.

Der Erfindung liegt die Aufgabe zugrunde, Wegwerfwindeln des zweiten Typs so zu verbessern, daß der vordere Abschnitt der Windel eine ausreichende Saugfähigkeit für die Flüssigkeit aufweist.

### ZUSAMMENFASSUNG DER ERFINDUNG

Die Aufgabe wird erfindungsgemäß bei einer Wegwerfwindel gemäß Anspruch 1 gelöst.

Bei einem bevorzugten Ausführungsbeispiel sind die Bauchabschnitte aus einer flüssigkeitsdurchlässigen oberen Lage, einer flüssigkeitsdurchlässigen rückwärtigen Lage und einem Flüssigkeitssaugkern hergestellt, der zwischen diesen beiden Lagen sandwichartig eingelegt ist.

Bei einem anderen bevorzugten Ausführungsbeispiel, insbesondere wenn die Bauchabschnitte ohne Saugkern ausgestattet and, dann wird eine Flüssigkeitssaugkapazität pro qm des Kerns in dem vorderen Abschnitt eingestellt, die höher ist als diejenige, in den anderen Abschnitten. Obwohl die Anlegefolge dem oben beschriebenen zweiten Typ entspricht, d.h. die Bauchabschnitte werden aufeinander gelegt und anschließend der vordere Abschnitt auf diese Bauchabschnitte gelegt, saugt erfindungsgemäß der Abschnitt des Flüssigkeitssaugkerns, der den vorderen Abschnitt einnimmt, in zufriedenstellender Weise, die Menge von flüssigen Ausscheidungen auf, die durch die Bauchabschnitte hindurch getreten sind, da die Bauchabschnitte flüssigkeitsdurchlässig sind.

Wenn die Bauchabschnitte ebenfalls mit einem flüssigkeitssaugenden Kern ausgestattet werden, so werden flüssige Ausscheidungen nicht nur durch den Abschnitt des Saugkerns aufgesaugt, der den vorderen Abschnitt einnimmt, sondern auch von dem Abschnitt des Sauggkerns, der in den Bauchabschnitten angeordnet ist.

Demzufolge wird der vordere Abschnitt wirksam zum Absaugen von Flüssigkeitsausscheidungen verwendet und das Problem des Stands der Technik gelöst, daß eine ungenügende Saugkapazität ein unerwünschtes Auslaufen der Ausscheidungen verursacht. Insbesondere wenn der Träger männlichen Geschlechts ist oder wenn bei einem Träger weiblichen Geschlechts dieser auf einer Seite im Bett liegt, wird eine genügende Aufsaugkapazität in dem Frontabschnitt sichergestellt.

Die Erfindung geht aus von dem oben erwähnten zweiten Typ einer konventionellen Wegwerfwindel für Erwachsene, die so gestaltet ist, daß beim Anlegen die Flügelabschnitte aufeinandergelegt und miteinander befestigt werden, um die Windel um die Hüfte des Trägers festzulegen und wobei anschließend der vordere Abschnitt auf diese Bauchabschnitte gelegt wird. Dadurch kann die Windel leicht und zuverlässig mit Hilfe einer Pflegeperson oder durch den Träger selber angelegt werden. Des weiteren kann die Pflegeperson durch einfaches Öffnen des vorderen Abschnitts prüfen, ob Ausscheidungen vorhanden sind, wobei die Bauchabschnitte miteinander befestigt bleiben, so daß die Windel aus ihrer ursprünglichen Position nicht verschoben wird, so daß nach dem Prüfen der vordere Abschnitt zurück auf die Bauchabschnitte gefaltet werden kann.

### KURZBESCHREIBUNG DER ZEICHNUNGEN

Es zeigen:
- Fig. 1: eine Ansicht einer Abwicklung mit teilweise weggebrochenen Bereichen eines erfindungsgemäßen Ausführungsbeispiels mit Sicht auf die Innenseite (Oberseite);
- Fig. 2: eine Ansicht ähnlich derjenigen in Fig. 1 mit Sicht auf die Außenseite (Unterseite);
- Fig. 3: eine Ansicht eines Schnitts englang der Linien III-III in Fig. 1;
- Fig. 4: eine perspektivische Ansicht einer Windel bei einem Zwischenschritt der Anlegefolge; und
- Fig. 5: eine ähnliche Ansicht wie diejenige in Fig. 1 eines weiteren Ausführungsbeispiels.

### BEVORZUGTE AUSFÜHRUNGSFORMEN

Im folgenden werden Ausführungbeispiele der Erfindung unter Bezugnahme auf die Zeichnungen näher erläutert.

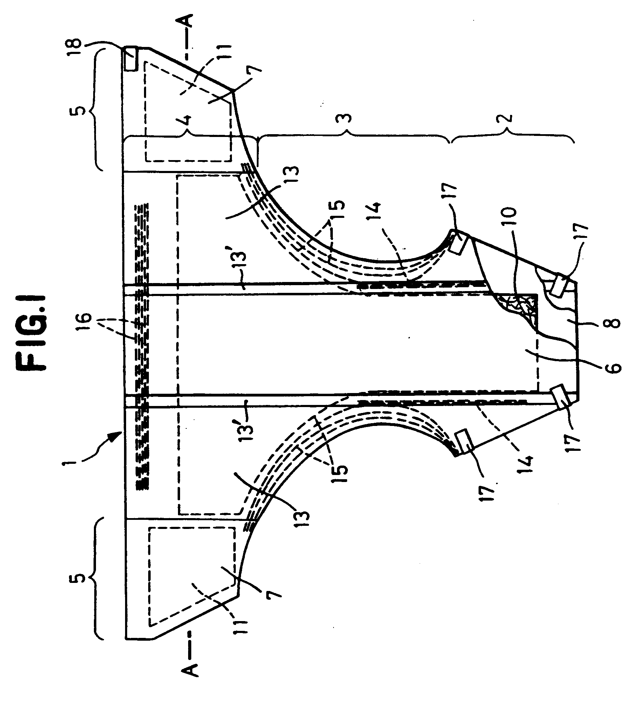

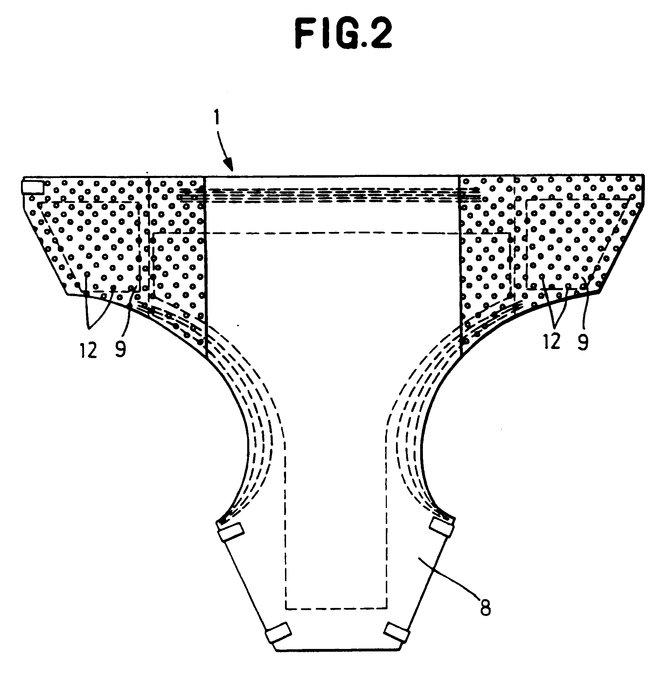

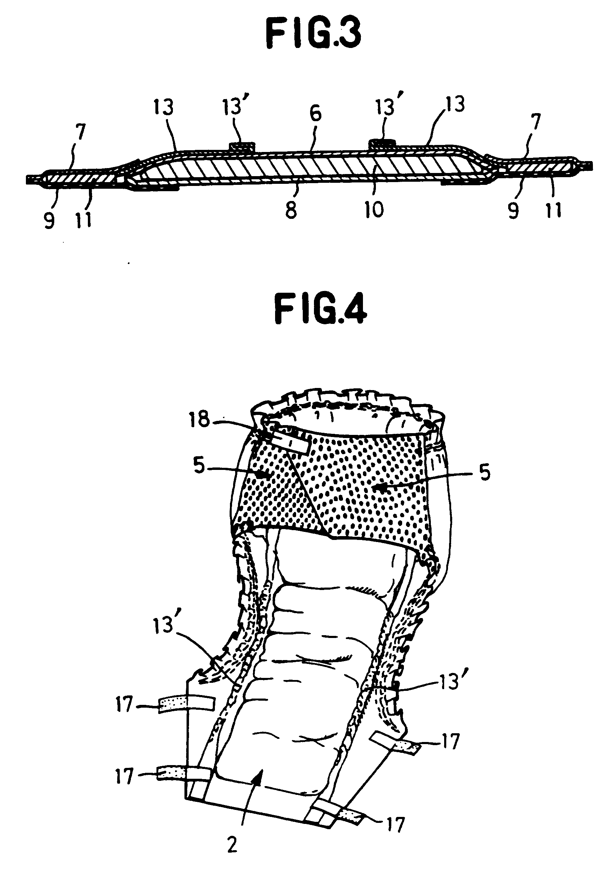

Es wird zunächst Bezug genommen auf die Figuren 1 bis 4. Eine Wegwerfwindel 1 besitzt einen vorderen Abschnitt 2, einen Schrittabschnitt 3, einen rückwärtigen Abschnitt 4 und ein Paar Bauchabschnitte 5, die sich seitlich von einander gegenüberliegenden Seiten des rückwärtigen Abschnitts 4 wegerstrecken. Die Windel 1 besteht aus einer ersten flüssigkeitsdurchlässigen oberen Lage 6, die den vorderen Abschnitt 2, den Schrittabschnitt 3 und den rückwärtigen Abschnitt 4 überdeckt, einer zweiten flüssigkeitsdurchlässigen oberen Lage 7, die die Bauchabschnitte 5 überdeckt, einer ersten flüssigkeitsundurchlässigen rückwärtigen Lage 8, die den vorderen Abschnitt 2, den Schrittabschnitt 3 und den rückwärtigen Abschnitt 4 überdeckt, einer zweiten flüssigkeitsdurchlässigen rückwärtigen Lage 9, die die Bauchabschnitte 5 bedeckt und einem ersten Flüssigkeitssaugkern 10, der zentral zwischen der ersten oberen Lage 6 und der ersten rückwärtigen Lage 8 sandwichartig eingelegt ist. Ein zweiter Flüssigkeitssaugkern 11 ist sandwichartig zwischen den jeweiligen zweiten oberen Lagen 7 und der entsprechenden zweiten rückwärtigen Lage 9 eingelegt. Jeder dieser zweiten Saugkerne 11 ist dünner als der erste Saugkern 10 und kann auch intelgral mit dem ersten Saugkern 10 ausgebildet sein, obwohl die zweiten Kerne 11 in der Zeichnung als von dem ersten Kern 10 getrennt ausgebildet dargestellt sind.

Die zweiten oberen Lagen 7 sind mit der ersten oberen Lage 6 durch zugeordnete Seitenlagen 13 und den zweiten rückwärtigen Lagen 9 jeweils unmittelbar mit der ersten rückwärtigen Lage 8 unter Verwendung eines geeigneten Klebemittels oder mittels Schweißmitteln verbunden. Die zweiten rückwärtigen Lagen 9 sind mit feinen Perforationen 12 ausgestattet. Seitlich einander gegenüberliegende Seiten der oberen und der rückwärtigen Lagen 6 und 8 und der erste Kern 10 sind entlang des Schrittabschnitts 3 nach innen gekrümmt ausgebildet. Der erste Kern 10 erstreckt sich seitlich in den rückwärtigen Abschnitt 4 und bildet im wesentlichen eine T-Form.

Auf der Oberseite einer Oberfläche der Windel 1, d.h. auf einer Oberfläche der oberen Lage 6, ist in einem in Längsrichtung zentralen Abschnitt ein Paar elastischer schmaler Seitenlagen 13 so angeordnet, daß sie an beiden Seiten des vorderen Abschnitts 2 einander gegenüberliegen. Der Schrittabschnitt 3 und der rückwärtige Abschnitt 4 und Außenseite der jeweiligen Seitenlagen 13 sind auf der Oberseite der oberen Lage 6 befestigt. Den Seitenlagen 13 ist durch elastische Teile 14 eine Elastizität verliehen, wobei die elastischen Teile 14 in Randkantenhüllen 13' der jeweiligen Seitenlagen 13 eingeschlossen sind. Obwohl die jeweiligen Seitenlagen 13 so dargestellt sind, daß die inneren Enden nach außen geklappt und auf die obere Lage 6 befestigt sind, ist es jedoch auch möglich, die inneren Enden nach innen zu klappen und sie an der Oberlage 5 zu befestigen oder ein Ende nach innen und das andere nach außen zu klappen und sie dann an der Oberlage 6 zu befestigen.

Die Windel 1 ist mit elastischen Teilen 15 versehen, die sich entlang einander gegenüberliegender Ränder im Schrittabschnitt 3 erstrecken. Des weiteren sind elastische Teile 16 vorgesehen, die sich entlang der oberen Kante des rückwärtigen Bereichs 4 erstrecken, wobei beide elastischen Teile 15,16 zwischen den oberen und unteren Lagen 6,8 sandwichartig eingeschlossen sind.

Die Windel 1 ist des weiteren an vier Ecken im vorderen Abschnitt 2 und an einer oberen Ecke an einem der Flügelabschnitte 5 mit Klebebändern 17 bzw. 18 ausgestattet.

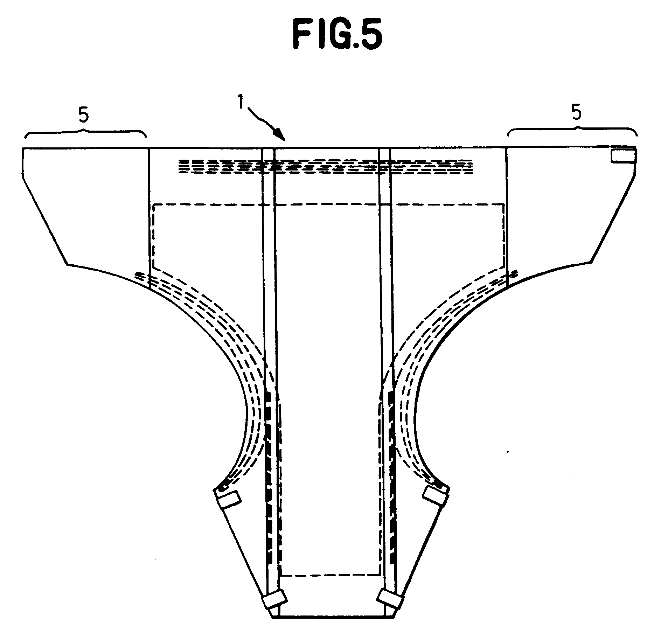

Es wird nun Bezug genommen auf Fig. 5. In dieser Figur ist ein weiteres Ausführungsbeispiel der Erfindung dargestellt, bei dem jedoch kein zweiter Kern 11 vorhanden ist. Der übrige Teil des Aufbaues ist ähnlich des vorher beschriebenen Ausführungsbeispiels. Bei dieser Ausführungsform ist es jedoch auch möglich, jeden der Flügelabschnitte aus einer einzelnen Lage einer porösen Lage herzustellen. Bei diesem Ausführungsbeispiel hat der erste Kern 10 vorzugsweise eine Flüssigkeitssaugkapazität pro qm, die im vorderen Abschnitt höher ist als in anderen Abschnitten, um eine ausreichende Saugkapazität bereitzustellen.

Es liegt auf der Hand, daß die Kontur der Windel 1 im vorderen Abschnitt 2 und in den Bauchabschnitten 5 anders gestaltet sein kann, als dargestellt.

Vorzugsweise besteht die erste und die zweite obere Lage 6,7 aus einem Faservlies, die erste rückwärtige Lage 8 aus einem Kunststoffilm, die zweite rückwärtige Lage 9 aus einem offenen Kunststoffilm oder einem offenen Faservlies, der erste und zweite Saugkern 10 und 11 aus einer flockigen Pulpe mit supersaugfähigen Polymerpartikeln und die Seitenlagen 13 bestehen wiederum aus einem Faservlies. Es kann jedes entsprechend geeignete Material verwendet werden.

Im folgenden wird eine Abfolge des Anlegevorgangs der erfindungsgemäßen Wegwerfwindel beschrieben. Die Pflegeperson plaziert den rückwärtigen Abschnitt 4 auf die rückwärtige Hüfte des Trägers, faltet dann die Bauchabschnitte 5 aufeinander, so daß der eine Bauchabschnitt 5 mit dem Klebeband 18, den anderen Bauchabschnitt 5 auf dem Bauch des Trägers überdeckt. Anschließend wird das Befestigungsband 18 an dem anderen Bauchabschnitt 5 angeklebt. Dann plaziert die Pflegeperson den Schrittabschnitt 3 auf den Schritt des Trägers und hält den vorderen Abschnitt 2 in der Hand, um ihn über den Bauch des Trägers zu legen und so die Bauchabschnitte 5 zu überdecken. Schließlich befestigt die Pflegeperson die Klebebänder 17 des vorderen Abschnitts an den Bauchabschnitten.

Wenn die erfindungsgemäße Windel von dem oder der Benutzer(in) selber angelegt wird, kann die Anlegefolge in der gleichen Weise von dem stehenden Träger durchgeführt werden.

## Patentansprüche

1. Wegwerfwindel mit folgenden Merkmalen:
eine flüssigkeitsdurchlässige obere Lage (6) überdeckt einen vorderen Abschnitt (2), einen Schrittabschnitt (3) und einen rückwärtigen Abschnitt (4),
eine flüssigkeitsundurchlässige rückwärtige Schicht (8) überdeckt diese Bereiche ebenfalls,
ein erster Flüssigkeits-Saugkern (10) ist zwischen der oberen Lage (6) und der rückwärtigen Schicht (8) sandwichartig eingelegt,
ein Paar Bauchabschnitte (5) erstrecken sich von einander gegenüberliegenden Seiten des rückwärtigen Bereichs (4) seitlich weg, so daß die Windel durch Befestigen der Bauchabschnitte (5) aneinander angelegt und anschließend der vordere Abschnitt (2) auf diese Bauchabschnitte (5) gelegt und an diesen befestigt werden kann, dadurch **gekennzeichnet**, daß die Bauchabschnitte (5) aus einem flüssigkeitsdurchlässigen Material hergestellt sind.

2. Wegwerfwindel nach Anspruch 1, wobei jeder der Bauchabschnitte (5) eine flüssigkeitsdurchlässige obere Lage (7), eine flüssigkeitsdurchlässige rückwärtige Lage (9) und einen Zweiten flüssigkeitsabsorbierenden Kern (11) aufweist, der zwischen den beiden genannten Lagen eingelegt ist.

3. Wegwerfwindel nach Anspruch 1 und 2, dadurch gekennzeichnet, daß statt den Flüssigkeits-Saugkern (11) zwischen den beiden Lagen jeder der Bauchabschnitte (5) anzuordnen, eine Flüssigkeits-Saugkapazität pro qm des Saugkerns (11) im vorderen Abschnitt (2) eingestellt wird, die höher ist als die Kapazität in anderen Abschnitten.

## Claims

1. A disposable diaper having the following features:
a liquid-permeable top layer (6) covers a front portion (2), a crotch portion (3) and a rear portion (4),
a liquid-impermeable rear layer (8) also covers these portions,
a first liquid-absorbent core (10) is inserted sandwich-like between the top layer (6) and the rear layer (8),
a pair of belly portions (5) extend sideways from opposite sides of the rear portion (4) so that the diaper can be put on by fixing the belly portions (5) to each other, and subsequently the front portion (2) can be laid upon and fixed to said belly portions (5), characterized in that the belly portions (5) are made of a liquid-permeable material.

2. A disposable diaper as claimed in claim 1, wherein each of the belly portions (5) has a liquid-permeable top layer (7), a liquid-permeable rear layer (9) and a second liquid-absorbent core (11) inserted between said layers.

3. A disposable diaper as claimed in claim 1 and 2, characterized in that instead of arranging the liquid-absorbent core (11) between the two layers of each of the belly portions (5), a liquid-absorption capacity per square meter of the absorbent core (11) in the front portion (2) is set which is higher than the capacity in the other portions.

## Revendications

1. Couche jetable avec les caractéristiques suivantes :
une couche supérieure (6) perméable aux liquides recouvre une partie antérieure (2), une partie pour les jambes (3) et une partie postérieure (4),
une couche arrière (8) imperméable aux liquides recouvre également ces zones,
un premier matelas intérieur (10) absorbant le liquide est intercalé en sandwich entre la couche supérieure (6) et la couche arrière (8),
deux parties ventrales (5) s'étendent sur les côtés en s'écartant des côtés opposés de la partie postérieure (4), de telle sorte que la couche peut être posée en fixant les parties ventrales (5) l'une contre l'autre et, ensuite, la partie antérieure (2) peut être rabattue sur lesdites parties ventrales (5) et être fixée contre celles-ci,
caractérisée en ce que les parties ventrales (5) sont réalisées en matériau perméable aux liquides.

2. Couche jetable selon la revendication 1, dans laquelle chacune des parties ventrales (5) comporte une couche supérieure (7) perméable aux liquides, une couche arrière (9) perméable aux liquides et un deuxième matelas intérieur (11) absorbant le liquide, lequel est intercalé entre les deux couches mentionnées.

3. Couche jetable selon les revendications 1 et 2, caractérisée en ce que, au lieu d'insérer le matelas intérieur (11) absorbant le liquide entre les deux couches de chaque partie ventrale (5), il est prévu de réguler une capacité d'absorption de liquide par m² du matelas d'absorption (11) dans la partie antérieure (2) qui est supérieure à la capacité dans les autres parties.
